# EUROPÄISCHE PATENTSCHRIFT

(11) **EP 3 538 517 B1**
(45) Veröffentlichungstag und Bekanntmachung des Hinweises auf die Patenterteilung: **10.06.2020**
(21) Anmeldenummer: 17797303.9
(22) Anmeldetag: 06.11.2017
(51) Int. Cl.: C07D 401/14, C07D 213/89, C07C 275/06, C07F 9/6518, C07F 9/6561, C07D 403/06, C07C 231/02, C07D 249/18, C07D 471/04, C07D 253/08, C07D 207/263, C07D 295/24, C07F 9/535

(54) **VERFAHREN ZUR SYNTHESE VON AMIDBINDUNGEN MIT HILFE NEUER KATALYSATOREN**
METHOD FOR SYNTHESISING AMIDE BONDS WITH THE AID OF NEW CATALYSTS
PROCÉDÉ DE SYNTHÈSE DE LIAISONS AMIDES À L'AIDE DE NOUVEAUX CATALYSEURS

(30) Priorität: 09.11.2016 EP 16197873
(43) Veröffentlichungstag der Anmeldung: 18.09.2019
(73) Patentinhaber: Merck Patent GmbH, 64293 Darmstadt (DE)
(72) Erfinder: IGNATYEV, Nikolai (Mykola), 47058 Duisburg (DE); FRANK, Walter, 42329 Wuppertal (DE); BARTHEN, Peter, 47495 Rheinberg (DE); VYSOTSKY, Myroslav, 55218 Ingelheim (DE)
(86) Internationale Anmeldenummer: PCT/EP2017/078261
(87) Internationale Veröffentlichungsnummer: WO 2018/087019

(56) Entgegenhaltungen:
- WO-A1-03/002579

## Beschreibung

Die Erfindung betrifft ein Verfahren zur Herstellung von Amidbindungen, insbesondere von Peptidbindungen, mit Hilfe neuer Amidknüpfungsreagenzien als Katalysatoren, die neuen Reagenzien und deren Herstellung.

Moderne Standardverfahren zur Peptidsynthese nutzen effektive Peptidknüpfungsreagenzien. Die Bildung von Amidbindungen ist ebenfalls für die Synthese anderer organischer Moleküle von Bedeutung, beispielsweise bei der Synthese der Verbindungen Atorvastatin, Lisinopril oder Diltiazem. Eine Amidbindung wird durch Reaktion einer Säure mit einem primären oder sekundären Amin in Gegenwart einer Base und mit Hilfe eines Amidknüpfungsreagenzes gebildet. Die Peptidsynthese ist, vereinfacht beschrieben, eine Reaktion zwischen einer Aminosäure und einem primären oder sekundären Amin in Gegenwart von einem Äquivalent Peptidknüpfungsreagenz und zwei Äquivalenten tertiärem Amin (als Protonenakzeptor) in zwei Schritten. Der erste Schritt ist die Aktivierung einer N-geschützten Aminosäure zum aktiven Ester, der im zweiten Schritt praktisch sofort mit einer Carboxyl-geschützten Aminosäure (Amin) zum Amid reagiert. Die Amidknüpfungsreagenzien bzw. Peptidknüpfungsreagenzien haben eine oder mehrere funktionelle Gruppen, die nicht nur zur Aktivierung einer Carboxylgruppe dienen, sondern auch zur Eliminierung von einem Äquivalent Wasser.

Die Geschichte der Peptidknüpfungsreagenzien beginnt mit Carbodiimiden, die nur eine Carbodiimid-Gruppe tragen. Dicyclohexylcarbodiimid z. B. wird seit 1955 untersucht und reagiert im ersten Schritt mit einer Carbonsäure zu einem O-Acyl-Harnstoff, welcher mit seiner sehr aktiven Ester-Gruppe mit dem Amin reagiert und dabei ein Äquivalent Harnstoff bildet, der sich normalerweise leicht abtrennen lässt. Es hat sich jedoch gezeigt, dass einerseits *N*-Acyl-Harnstoff als Nebenprodukt entsteht, andererseits solche Peptidknüpfungsreaktionen allgemein unter starker Epimerisierung (Racemisierung, Konfigurationsumkehr) ablaufen. Modernere Peptidknüpfungsreagenzien erlauben die Knüpfung von einem Äquivalent Säure und einem Äquivalent Amin mit viel geringerem Epimerisierungsgrad und tragen eine positiv geladene Gruppe, die während der Reaktion zu beispielsweise Harnstoff oder zu Phosphoroxid reagiert und eine neutrale Gruppe, die nur zur Aktivierung einer Säuregruppe dient. Solche effizienten Peptidknüpfungsreagenzien liegen als organische Salze, z.B. Uronium- [F. Albericio, J. M. Bofill, A. El-faham, S. A. Kates, J. Org. Chem. 1998, 63, 9678-9683], Aminium- [L. A. Carpino, H. Imazumi, A. El-faham, F. J. Ferrer, C. W. Zhang, Y. S. Lee, B. M. Foxman, P. Henklein, C. Hanay, C. Mugge, H. Wenschuh, K. Klose, M. Beyermann, M. Bienert, Angew. Chem. Int. Ed. 2002, 41, 442-445], Immonium- (bzw. Carbonium-) [P. Li, J. C. Xu, Tetrahedron 2000, 56, 4437-4445] [P. Li, J. C. Xu, J. Pept. Res. 2001, 58, 129-139], Imidazolium- [Anderson, G. W.; Paul, R. J. Am. Chem. Soc. 1958, 80, 4423] [A. K. Saha, P. Schultz, H. Rapoport, J. Am. Chem. Soc. 1989, 111, 4856-4859] [F. S. Gibson, H. Rapoport, J. Org. Chem. 1995, 60, 2615-2617] oder Phosphonium-Salze [B. Castro, J. R. Dormoy, G. Evin and C. Selve, Tetrahedron Lett. 1975, 14, 1219-1222] [F. Albericio, J. M. Bofill, A. El-Faham and S. A. Kates, J. Org. Chem. 1998, 63, 9678-9683] [J. Coste, D. Le-Nguyen, B. Castro, Tetrahedron Lett. 1990, 31, 205-208] [F. Albericio, M. Cases, J. Alsina, S. A. Triolo, L. A. Carpino, S. A. Kates, Tetrahedron Lett. 1997, 38, 4853-4856] [T. Hoeg-Jensen, C. E. Olsen and A. Holm, J. Org. Chem. 1994, 59, 1257-1263] vor. Neuere Fluoruronium-Peptidknüpfungsreagenzien nutzen die Bildung von Säurefluoriden bei der Reaktion mit Aminosäuren. Besonders effektiv werden hier Fluor-*N,N,N',N'-*tetramethylformamidinium-hexafluorophosphat (TFFH) und Bis(tetramethylen)fluorformamidiniumhexafluorophosphat (BTFFH) [L. A. Carpino, A. Elfaham, J. Am. Chem. Soc. 1995, 117, 5401-5402] [A. El-Faham, Chem. Lett. 1998, 671-672] eingesetzt.

Neueste und vielfältig untersuchte Peptidknüpfungsreagenzien werden als nicht explosiver Ersatz für Benzotriazol-Derivate eingesetzt, z. B. Ethyl(hydroxyimino)cyanoactetat (Oxyma) und, mit dem zusätzlichen Vorteil des positiven Einflusses eines Morpholin-Substituenten, (1-Cyano-2-ethoxy-2-oxoethylidenaminooxy)-dimethylamino-morpholinocarbeniumhexafluorophosphat (COMU) [L. Spanier, E. Ciglia, F. K. Hansen, K. Kuna, W. Frank, H. Gohlke, T. Kurz, J. Org. Chem. 2014, 79, 1582-1593].

Dabei ist besonders das Verhalten der Amidknüpfungsreagenzien, insbesondere der Peptidknüpfungsreagenzien, in Lösung von aktuellem Interesse. Die Gegenionen der zur Zeit eingesetzten Reagenzien sind in der Regel Tetrafluoroborat oder Hexafluorophosphat. Die Löslichkeit der bisher bekannten Amidknüpfungsreagenzien zur Herstellung von Amidbindungen in organischen Lösemitteln ist nach wie vor unbefriedigend bzw. die Auswahl eines geeigneten Lösemittels für die gewünschte Synthese der Amidbindung ist oftmals beschränkt, was in der Regel zu geringen Umsätzen bei der Reaktion führt.

Es ist aus dem Stand der Technik beispielsweise bekannt, dass eine Fluorierung durch die Wahl von unterschiedlichen Anionen im Fluorierungsreagenz beeinflusst werden kann. In WO 2011/124307 wird beschrieben, dass Fluorierungsreagenzien mit Perfluoralkylfluorophosphat-Anionen vorteilhafte Eigenschaften hinsichtlich ihrer Löslichkeit, Stabilität und Reaktivität im Vergleich zu Fluorierungsreagenzien mit anderen Anionen zeigen. Das Anion scheint bei dieser Reaktion von entscheidender Bedeutung zu sein. Es wird angenommen, dass die Stärke der elektrostatischen Wechselwirkung zwischen Anion und Kation auch hier die Reaktivität des Kations beeinflusst, wie dies für einige andere Reaktionen ebenfalls beschrieben ist [I. Krossing, I. Raabe, Angew. Chem. 2004, 116, 2116-2142] [M. Uyanik, D. Nakashima, K. Ishihara, Angew. Chem. 2012, 124, 9227-9230].

Aus WO 03/002579 sind Säuren und Salze mit Fluoralkylfluorophosphat-Anionen bekannt sowie deren Verwendung in einem Verfahren zur Carboaminierung oder Carboamidierung von Olefinen.

Bis heute hat man jedoch keine wesentlichen Reaktivitätsunterschiede für Peptidknüpfungsreagenzien mit gleichem Kation und unterschiedlichen Anionen beobachtet [V. Dourtoglou, J.-C. Ziegler, B. Gross, Tetrahedron Lett. 1978, 15, 1269-1272] [R. Knorr, A. Trzeciak, W. Bannwarth, D. Gillessen, Tetrahedron Lett. 1989, 30, 1927-1930].

Wie man in dem allgemeinen Schema mit dem Beispiel *O*-(3,4-Dihydro-4-oxo-1,2,3-benzotriazin-3-yl)-*N*,*N*,*N*',*N*'-tetramethyluroniumtetrafluoroborat (TDBTU) als Amidknüpfungsreagenz sieht, gibt es mehrere Schritte bei der Säureaktivierung:

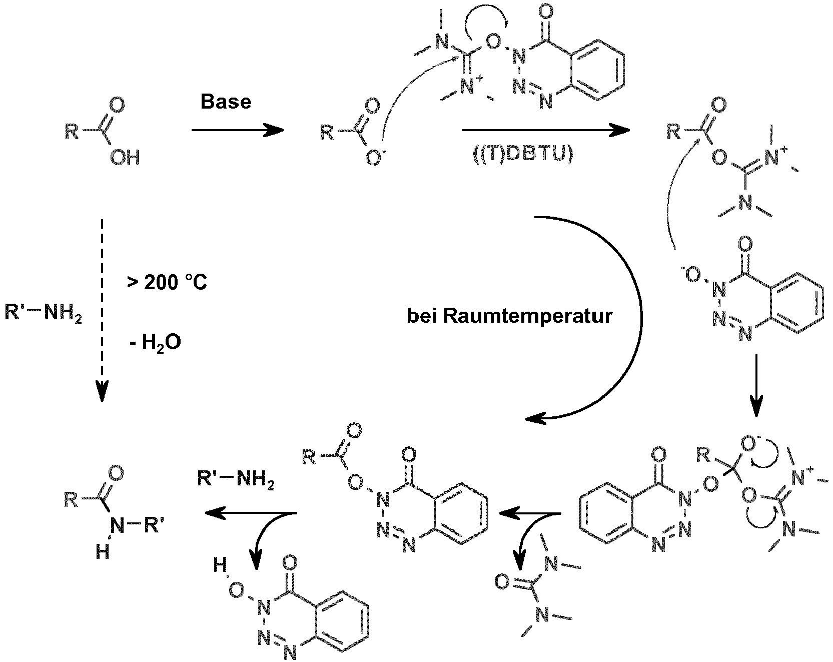

Die Carbonsäure (RCOOH) wird zunächst durch die Base deprotoniert und anschließend reagiert das entstandene Anion mit dem Kation des Amidknüpfungsreagenzes. Das entstehende Anion greift nachfolgend den Carbonylkohlenstoff der Carbonsäure an, wobei ein Molekül Tetramethylharnstoff entsteht und ein aktiver Ester, der mit dem Amin (R'-NH₂) zum Amid (R-C(O)-NH-R') reagiert. Bei dieser Reaktion laufen zwei Schritte ab, für die die Reaktivität der positiv geladenen Gruppen eine entscheidende Rolle spielt. Um die Reaktivität zu verbessern, hat sich eine möglichst schwache Koordination des Kations des Amidknüpfungsreagenzes an das Gegenanion bewährt, d.h. die Anwendung schwach koordinierender Anionen ist vorteilhaft.

Die Kupplungsreaktion zur Amidbindung sollte unter idealen Bedingungen in hoher Geschwindigkeit ohne Racemisierung und Nebenreaktionen und in hoher Ausbeute bei Einsatz äquimolarer Mengen an Carboxy- und Aminokomponente ablaufen.

Nach wie vor besteht ein Bedarf an neuen Amidknüpfungsreagenzien, die möglichst nahe an diese idealen Bedingungen heranreichen und dem Synthesefachman eine größere Flexiblität bei der richtigen Auswahl an Reagenzien und Reaktionsbedingungen bieten.

Die Aufgabe der vorliegenden Erfindung war somit das Bereitstellen alternativer Amidknüpfungsreagenzien mit verbesserten Eigenschaften, z.B. bezüglich ihrer Kation-Anion-Wechselwirkung, Löslichkeit und/oder Reaktivität. Insbesondere soll dies eine erhöhte Variabilität hinsichtlich geeigneter Lösemittel für die Amidknüpfungsreaktion zur Folge haben. Es ist ebenfalls Aufgabe der Erfindung, die Amidbindungsbildung zu beschleunigen und/oder die Umsätze und Ausbeute zu erhöhen.

Überraschenderweise wurde nun gefunden, dass Amidknüpfungsreagenzien enthaltend Perfluoralkylfluorophosphat-Anionen diese Aufgabe erfüllen.

Die neuen Verbindungen zeigen eine deutlich höhere Löslichkeit in vielen organischen Lösemitteln, besonders in Dichlormethan, Acetonitril, Tetrahydrofuran (THF), Dimethylformamid (DMF) oder deren Gemischen, als die entsprechenden Tetrafluoroborate oder Hexafluorophosphate. Diese verbesserte Löslichkeit ermöglicht eine hohe Variabilität an geeigneten Lösemitteln für die gewünschte Anwendung in der Amidknüpfungsreaktion. Durch die neuen Reagenzien können daher auch Säuren und Amine miteinander verknpüft werden, die in den Standard-Lösemitteln bisher nicht oder nur mit unbefriedigender Ausbeute reagieren konnten.
Die neuen Amidknüpfungsreagenzien enthaltend Perfluoralkylfluorophosphat-Anionen können weiterhin *in situ* aus entsprechenden Amidknüpfungsreagenzien mit [BF₄]- oder [PF₆]-Anion und Metathese mit einem entsprechenden Kalium oder Natrium Perfluoralkylfluorophosphat generiert werden.

Die Untersuchungen zur Säureaktivierung von Z-Aib-OH (Z-geschützte Aminoisobuttersäure) als Testsubstanz in THF als Lösemittel und in der Gegenwart von Collidin (TMP) als Base belegen in den meisten Fällen die höhere Aktivität der neuen Amidknüpfungsreagenzien im Vergleich zu den analogen Hexafluorophosphaten und Tetrafluoroboraten. Z-Aib-OH wurde für die Aktivierungsexperimente ausgewählt, weil die Verbindung eine Carboxylgruppe trägt, die nicht leicht zu aktivieren ist und die Reaktion leicht anhand von NMR-Experimenten verfolgt werden kann [L. A. Carpino, A. El-Faham, J. Org. Chem. 1994, 59, 695-698].

Die Synthesen von Dipeptiden am Beispiel von Ac-Phe-Ala-OMe zeigen höhere Umsätze und in einigen Fällen niedrigere Epimerisierungsgrade in Gegenwart der Perfluoralkylfluorophosphat-Verbindungen im Vergleich zu den analogen Hexafluorophosphaten und Tetrafluoroboraten. Nähere Erläuterungen werden im Ausführungsteil nachfolgend beschrieben. Der Vorteil der neuen Peptidknüpfungsreaktionen ist insbesondere der Zeitgewinn bei der Peptidsynthese durch schnellere Reaktionszeiten. Ein erster Gegenstand der Erfindung ist daher ein Verfahren zur Herstellung einer Amidbindung durch Reaktion einer Säure mit einem primären oder sekundären Amin in Gegenwart einer Base mit Hilfe eines Amidknüpfungsreagenzes, dadurch gekennzeichnet, dass mindestens ein Amidknüpfungsreagenz enthaltend ein Anion der Formel (I) verwendet wird,

[P(CₙF₂ₙ₊₁)_{y}F_{6-y}]⁻ (I),

wobei
n bei jedem Auftreten unabhängig für 1, 2, 3, 4, 5, 6, 7 oder 8 steht und y für 1, 2, 3 oder 4 steht.

Das Anion der Formel (I) kann auch mit der Abkürzung FAP⁻ beschrieben werden.

In einer bevorzugten Ausführungsform des Verfahrens werden Amidknüpfungsreagenzien verwendet, in denen die Variable n in dem Anion der Formel (I) bei jedem Auftreten unabhängig für die ganze Zahl 2, 3 oder 4 steht. Bevorzugt sind demzufolge Perfluoralkylfluorophosphat-Anionen der Formel (I), in denen die Perfluoralkylgruppen jeweils unabhängig voneinander 2, 3 oder 4 C-Atome haben. Besonders bevorzugt ist die Perfluoralkylgruppe in den Anionen der Formel (I) gleich. Geeignete Perfluoralkylgruppen mit 2 bis 4 C-Atomen sind demzufolge Pentafluorethyl, *n*- oder *iso*-Heptafluorpropyl und *n-, sec-, iso-* oder *tert-*Nonafluorbutyl.

Ein weiterer Gegenstand der Erfindung ist daher das Verfahren, wie zuvor beschrieben, wobei Amidknüpfungsreagenzien enthaltend Anionen der Formel (I) verwendet werden, in denen die Variable n bei jedem Auftreten unabhängig für 2, 3 oder 4 steht.

Bevorzugte Anionen der Formel (I) können aus der Gruppe der Anionen [(C₂F₅)₃PF₃]⁻, [(C₃F₇)₃PF₃]⁻, [(C₄F₉)₃PF₃]⁻, [(C₂F₅)₂PF₄]⁻, [(C₃F₇)₂PF₄]⁻, [(C₄F₉)₂PF₄]⁻, [(C₂F₅)PF₅]⁻, [(C₃F₇)PF₅]⁻ und [(C₄F₉)PF₅]⁻ ausgewählt werden. Die Perfluoralkylgruppen im Anion der Formel (I) sind bevorzugt geradkettig. Ganz besonders bevorzugte Anionen der Formel (I) sind [(C₂F₅)₃PF₃]⁻ und [(*n*-C₄F₉)₃PF₃]⁻.

Ein weiterer Gegenstand der Erfindung ist daher das Verfahren, wie zuvor beschrieben, wobei Amidknüpfungsreagenzien enthaltend Anionen der Formel (I) verwendet werden, in denen das Anion (I) aus der Gruppe [(C₂F₅)₃PF₃]⁻, [(C₃F₇)₃PF₃]⁻, [(C₄F₉)₃PF₃]⁻, [(C₂F₅)₂PF₄]⁻, [(C₃F₇)₂PF₄]⁻, [(C₄F₉)₂PF₄]⁻, [(C₂F₅)PF₅]⁻, [(C₃F₇)PF₅]⁻ und [(C₄F₉)PF₅]⁻ ausgewählt wird.

Das Kation der Amidknüpfungsreagenzien ist hierbei nicht eingeschränkt, sondern umfasst alle Kationen, die mit der entsprechenden Säure in dem erfindungsgemäßen Verfahren reagieren können, und diese entsprechend aktivieren.

Bevorzugte Kationen der Amidknüpfungsreagenzien im erfindungsgemäßen Verfahren werden aus der Gruppe Uronium-, Thiouronium-, Guanidinium-, Aminium-, Carbonium-, Imidazolium- und Phosphonium-Kationen ausgewählt.

Ein weiterer Gegenstand der Erfindung ist daher das Verfahren, wie zuvor beschrieben oder als bevorzugt beschrieben, wobei das Kation des Amidknüpfungsreagenzes ein Uronium-, ein Thiouronium-, ein Guanidinium-, ein Aminium-, ein Carbonium-, ein Imidazolium- oder ein Phosphonium-Kation bedeutet.

Das erfindungsgemäße Verfahren ist bevorzugt ein Verfahren zur Peptidherstellung und das beschriebene Amidknüpfungsreagenz ist bevorzugt ein Peptidknüpfungsreagenz.

Ein weiterer Gegenstand der Erfindung ist daher das Verfahren wie zuvor beschrieben oder bevorzugt beschrieben, wobei eine Peptidbindung hergestellt wird.

Bevorzugte Amidknüpfungs- bzw. Peptidknüpfungsreagenzien leiten sich von bekannten Peptidknüpfungsreagenzien ab, wobei jedoch erfindungsgemäß das Anion gegen ein Perfluoralkylfluorophosphat-Anion ausgetauscht wird.
Bekannte Peptidknüpfungsreagenzien sind
BTFFH bedeutend Bis(tetramethylen)fluorformamidiniumhexafluorophosphat,
BOP bedeutend (Benzotriazol-1-yloxy)tris(dimethylamino)phosphoniumhexafluorophosphat,
COMU bedeutend (1-Cyano-2-ethoxy-2-oxoethylidenaminooxy)-dimethylamino-morpholino-carbenium-hexafluorophosphat,
HATU bedeutend 1-(Dimethylamino(dimethylamonium-1-ylidene)methyl)-1*H*-azabenzo[*d*][1,2,3]triazol-3-oxid-hexafluorophosphat (käuflich als *O*-(7-azabenzotriazol-1-yl)-*N*,*N*,*N*',*N'*-tetramethyluronium-hexafluorophosphat bezeichnet),
HBPyU bedeutend 1-(Pyrrolidino(pyrrolidinium-1-ylidene)methyl)-1*H-*benzo[*d*][1,2,3]triazol-3-oxid-hexafluorophosphat (käuflich als *O-*(benzotriazol-1-yl)-*N*,*N*,*N'*,*N'*-bis(tetramethylene)uroniumhexafluorophosphat bezeichnet),
HBTU bedeutend 1-(Dimethylamino(dimethylamonium-1-ylidene)methyl)-1H-benzo[*d*][1,2,3]triazol3-oxid-hexafluorophosphat (käuflich als *O-*(benzotriazol-1-yl)-*N*,*N*,*N*',*N'*-tetramethyluronium-hexafluorophosphat bezeichnet),
HOTT bedeutend *S*-(1-Oxido-2-pyridyl)-*N*,*N*,*N*;*N'*-tetramethylthiouroniumhexafluorophosphat,
PfTU bedeutend *O*-Pentafluorophenyl-*N*,*N*,*N'*,*N'*-tetramethyluroniumhexafluorophosphat,
PyAOP bedeutend (7-Azabenzotriazol-1-yloxy)tris(pyrrolidino)phosphonium-hexafluorophosphat,
PyBOP bedeutend (Benzotriazol-1-yloxy)tris(pyrrolidino)phosphoniumhexafluorophosphat,
TDBTU bedeutend *O*-(3,4-Dihydro-4-oxo-1,2,3-benzotriazin-3-yl)-*N*,*N*,*N'*,*N'-*tetramethyluronium-tetrafluoroborat,
TFFH bedeutend Fluor-*N*,*N*,*N'*,*N'*-tetramethylformamidiniumhexafluorophosphat,
TSTU bedeutend *O*-(*N*-Succinimidyl)-*N*,*N*,*N*',*N'*-tetramethyluroniumtetrafluoroborat.

In dem erfindungsgemäßen Verfahren, wie zuvor beschrieben, werden daher besonders bevorzugt die folgenden Verbindungen als Amidknüpfungsreagenz oder Peptidknüpfungsreagenz verwendet, wobei FAP⁻ für das Anion der Formel (I) steht, wie zuvor beschrieben:

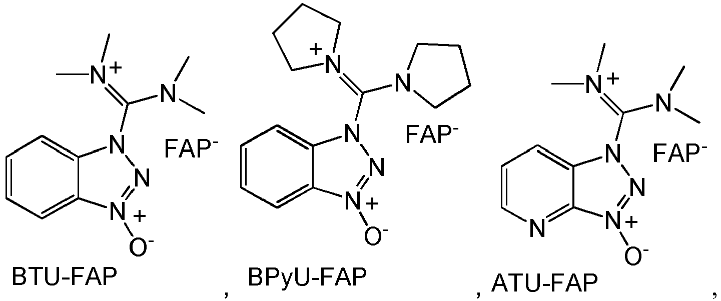

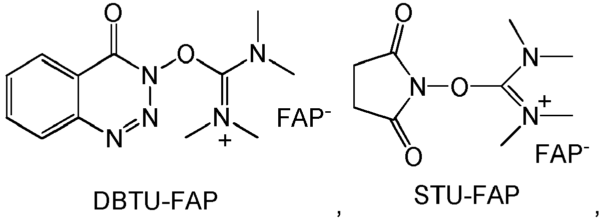

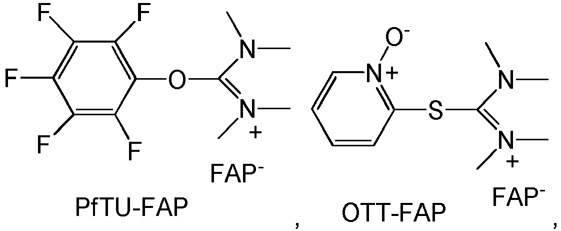

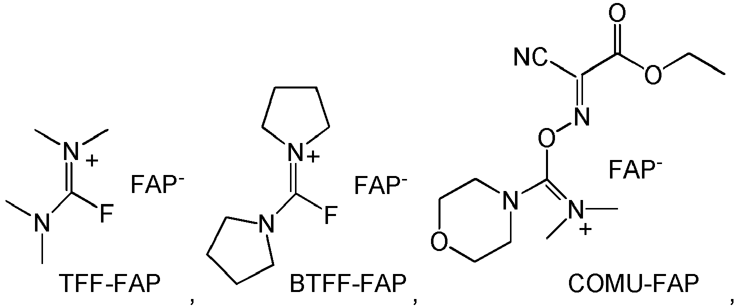

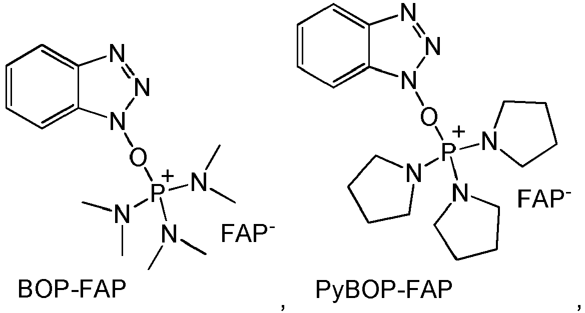

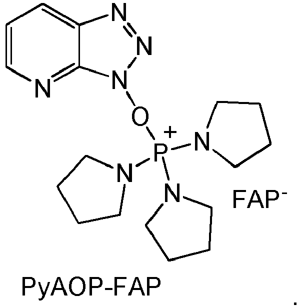

In dem erfindungsgemäßen Verfahren, wie zuvor beschrieben, werden daher ganz besonders bevorzugt die Verbindungen BTU-FAP, BPyU-FAP, ATU-FAP, DBTU-FAP, STU-FAP, PfTU-FAP, OTT-FAP, TFF-FAP, BTFF-FAP, COMU-FAP, BOP-FAP, PyBOP-FAP oder PyAOP-FAP als Amidknüpfungsreagenz oder Peptidknüpfungsreagenz verwendet, wobei FAP⁻ für ein bevorzugtes Anion der Formel (I) steht, wie zuvor beschrieben, insbesondere für [(C₂F₅)₃PF₃]⁻, [(C₃F₇)₃PF₃]⁻, [(C₄F₉)₃PF₃]⁻, [(C₂F₅)₂PF₄]⁻, [(C₃F₇)₂PF₄]⁻, [(C₄F₉)₂PF₄]⁻, [(C₂F₅)PF₅]⁻, [(C₃F₇)PF₅]⁻ und [(C₄F₉)PF₅]⁻bzw. ganz besonders bevorzugt für [(C₂F₅)₃PF₃]⁻ und [(*n*-C₄F₉)₃PF₃]⁻.

Aus der Gruppe der Amidknüpfungsreagenzien BTU-FAP, BPyU-FAP, ATU-FAP, DBTU-FAP, STU-FAP, PfTU-FAP, OTT-FAP, TFF-FAP, BTFF-FAP, COMU-FAP, BOP-FAP, PyBOP-FAP und PyAOP-FAP sind insbesondere die Amidknüpfungsreagenzien BTU-FAP, BPyU-FAP, ATU-FAP, DBTU-FAP, STU-FAP, PfTU-FAP, OTT-FAP, TFF-FAP, BTFF-FAP, COMU-FAP und BOP-FAP bevorzugt, wobei FAP⁻ für ein Anion der Formel (I) steht, wie zuvor beschrieben oder bevorzugt beschrieben.

Ein weiterer Gegenstand der Erfindung sind die Verbindungen BTU-FAP, BPyU-FAP, ATU-FAP, DBTU-FAP, STU-FAP, PfTU-FAP, OTT-FAP, TFF-FAP, BTFF-FAP, COMU-FAP, BOP-FAP, PyBOP-FAP und PyAOP-FAP, 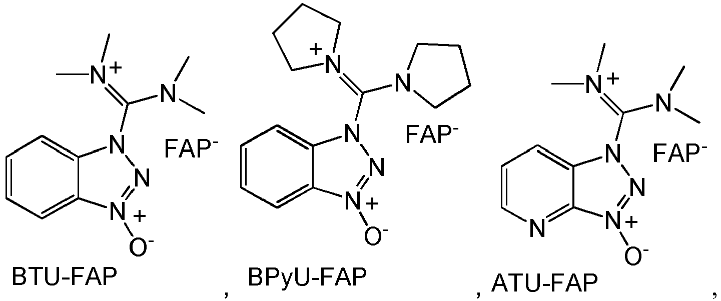 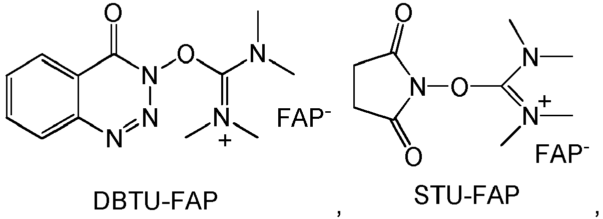 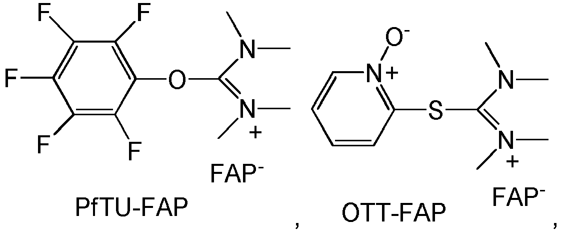 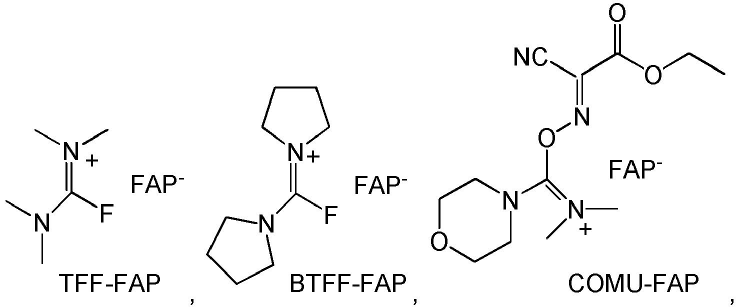 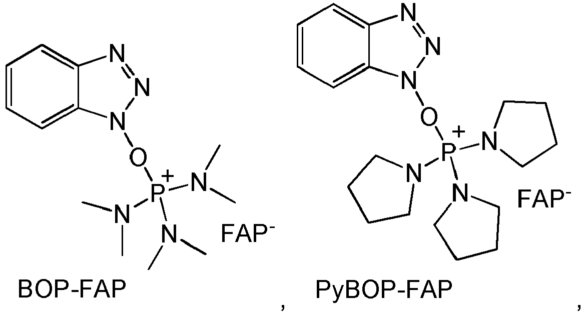 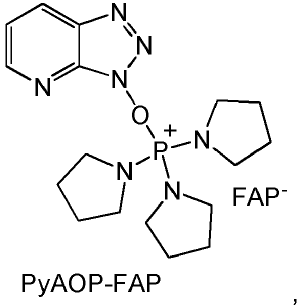 wobei FAP⁻ für ein Anion der Formel (I) steht, wie zuvor beschrieben oder für eine bevorzugte Ausführungsform von FAP⁻, wie zuvor beschrieben.

Ein weiterer Gegenstand der Erfindung sind die Verbindungen BTU-FAP, BPyU-FAP, ATU-FAP, DBTU-FAP, STU-FAP, PfTU-FAP, FAPOTT, TFF-FAP, BTFF-FAP, COMU-FAP, BOP-FAP, PyBOP-FAP und PyAOP-FAP, wobei FAP⁻ für [(C₂F₅)₃PF₃]⁻ und [(*n*-C₄F₉)₃PF₃]⁻ steht.

Die erfindungsgemäßen Verbindungen werden bevorzugt durch eine Metathesereaktion hergestellt, wobei die kommerziell erhältlichen Peptidknüpfungsreagenzien mit einem geeigneten Salz enthaltend das Anion der Formel (I) umgesetzt wird.

Geeignete Salze enthaltend das Anion der Formel (I) oder enthaltend eines der bevorzugten Anionen der Formel (I), wie zuvor beschrieben, sind Alkalimetallsalze. Bevorzugte Salze für die Metathese sind Natrium-, Kalium- oder Rubidiumsalze. Bevorzugte Salze sind Kaliumsalze enthaltend ein Anion der Formel (I) oder eine bevorzugte Ausführungsform des Anions der Formel (I).

Alternativ, können die Peptidknüpfungsreagenzien, wie zuvor beschrieben, in einer zweistufigen Synthese hergestellt werden, wie im folgenden Schema gezeigt:

| | |
|---|---|
| 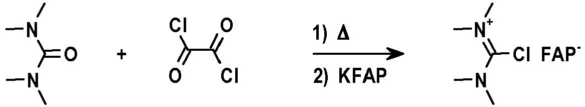 | Ausbeute 75% |
| 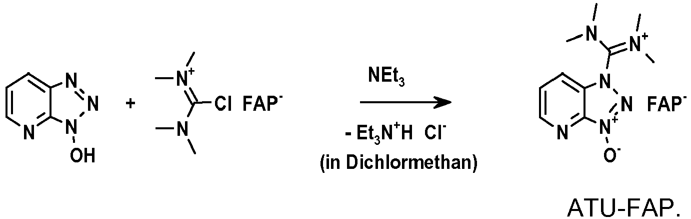 | Ausbeute 73% |

Die Metathese zur Herstellung der Amidknüpfungsreagenzien, wie zuvor beschrieben oder als bevorzugt beschrieben, kann in Wasser oder in organischen Lösemitteln oder einer Mischung dieser Lösemittel durchgeführt werden. Bevorzugt wird die Metathese in organischen Lösemitteln durchgeführt, bevorzugt bei Temperaturen zwischen -80° und 100°C, besonders bevorzugt bei -30°C bis Raumtemperatur. Geeignete organische Lösemittel werden beispielsweise ausgewählt aus Acetonitril, Propionitril, Aceton, Dioxan, Dichlormethan, Dimethoxyethan, Diethylether, Methyl-*t*-Butylether, Dimethylsulfoxid, Tetrahydrofuran, Dimethylformamid, *N,N*-Dimethylacetamid oder Alkohol, beispielsweise Methanol, Ethanol oder Isopropanol. Bevorzugt wird in den Lösemitteln Acetonitril, Propionitril, Aceton, oder einem Gemisch aus Acetonitril/Dichlormethan oder Acetonitril/Diethylether umgesetzt. Besonders bevorzugt wird in Acetonitril umgesetzt. Zwei bevorzugte Methoden der Metathese werden im Ausführungsteil beschrieben. Die Aufarbeitung erfolgt bevorzugt durch Entfernen des organischen Lösemittels und nachfolgende Extraktion, wobei gegebenenfalls vor Entfernung des Lösemittels unlösliche Bestandteile abfiltriert werden.

Wie zuvor beschrieben, ist die Auswahl an Säuren und primären oder sekundären Aminen für das erfindungsgemäße Verfahren und somit die Amidknüpfungsreaktion nicht eingeschränkt.
Viele Ausgangsmaterialien, wie zuvor beschrieben, sind kommerziell erhältlich oder werden nach Verfahren hergestellt, die dem Fachmann bekannt sind.

Besonders geeignete Ausgangsmaterialien für das erfindungsgemäße Verfahren sind entsprechend mit Schutzgruppen versehene Aminosäuren, die sowohl die Säure, als auch das primäre oder sekundäre Amin sein können. Die Art der Aminosäure ist hierbei nicht eingeschränkt. Sie können natürlichen oder auch synthetischen Ursprungs sein.
Die entsprechende Wahl der Schutzgruppe für die gewünschte Amidbildung ist dem Synthesefachmann bekannt. Eine vielfach verwendete Aminoschutzgruppe ist die Benzyloxycarbonyl-Gruppe, die auch mit der Abkürzung "Z" beschrieben wird. Es sind jedoch auch Acetyl-Gruppen geeignet, die oft mit der Abkürzung "Ac" beschrieben werden.

Im erfindungsgemäßen Verfahren ist die Base ebenfalls nicht eingeschränkt. Jede Art von Protonenakzeptoren ist möglich, um der Säure im ersten Schritt ein Proton zu entziehen, wie zuvor im allgemeinen Schema beschrieben. Viele Basen, wie zuvor beschrieben, sind kommerziell erhältlich. Bevorzugte Basen sind tertiäre Amine. Ein bekanntes Beispiel für ein tertiäres Amin, das als Base verwendet wird, ist 2,4,6-Trimethylpyridin, auch als Collidin oder TMP bezeichnet. Die Base wird vorzugsweise im Überschuss zugesetzt, bezogen auf die Menge der Säure. Es ist bevorzugt, drei Äquivalente an Base bezogen auf ein Äquivalent der Säure zu verwenden. Es ist weiter bevorzugt, jeweils ein Äquivalent an Amin und ein Äquivalent des Amidknüpfungsreagenzes zu verwenden, wie zuvor beschrieben.

Die weiteren Reaktionsbedingungen für eine Amidknüpfungsreaktion sind dem Synthesefachmann bekannt.
In einer bevorzugten Ausführungsform des erfindungsgemäßen Verfahrens erfolgt die Reaktion in einem organischen Lösemittel. Hierfür kann jedes gängige organische Lösemittel eingesetzt werden, in dem sich die zuvor beschriebenen neuen Amidknüpfungsreagenzien sowie die Säure und das Amin ausreichend lösen. Besonders geeignete organische Lösungsmittel sind Dichlormethan, Acetonitril, Tetrahydrofuran (THF), Dimethylformamid (DMF) oder deren Mischungen in allen Verhältnissen.
Besonders bevorzugt wird Tetrahydrofuran eingesetzt.

Im erfindungsgemäßen Verfahren erfolgt die Reaktion bevorzugt bei einer Temperatur zwischen -20°C bis 120°C, besonders bevorzugt ist eine Temperatur von 10°C bis 40°C. Ganz besonders vorteilhaft wird die Reaktion bei Raumtemperatur durchgeführt. Die Auswahl einer geeigneten Reaktionstemperatur kann von dem Fachmann ohne Weiteres getroffen werden.

Der eigentlichen Amidbindungsreaktion kann sich ein oder mehrere Aufreinigungsschritt(e) anschließen. Geeignete Aufreinigungsschritte umfassen das Abtrennen von leicht flüchtigen Komponenten durch Destillation oder Kondensation, eine Extraktion mit einem organischen Lösemittel, eine Fällung durch Zugabe eines organischen Lösemittels, einer Umsalzung oder eine Kombination dieser Methoden. Jede bekannte Trenn- bzw. Aufreinigungsmethode kann hierfür verwendet oder kombiniert werden.

Die folgenden Beispiele erläutern die vorliegende Erfindung näher, ohne den Schutzbereich zu beschränken. Insbesondere sind die in den Beispielen beschriebenen Reaktionsbedingungen, Merkmale, Eigenschaften und Vorteile der den betreffenden Beispielen zugrunde liegenden Amidknüpfungsreagenzien auch auf andere nicht im Detail ausgeführte, aber unter den Schutzbereich fallende Verfahren und Amidknüpfungsreagenzien anwendbar, sofern an anderer Stelle nicht Gegenteiliges gesagt wird. Im Übrigen ist die Erfindung im gesamten beanspruchten Bereich ausführbar und nicht auf die hier genannten Beispiele beschränkt.

NMR Spektroskopie: NMR-Proben werden entweder in einem 5 mm (Ø_{A}) Glas-NMR-Röhrchen oder in einem 3,7 mm (Ø_{A}) FEP-Inliner bei 25 °C gemessen. Bei Messungen in FEP wird der Inliner in ein 5 mm (Ø_{A}) Präzisions-Dünnglas-NMR-Röhrchen (Wilmad 537) eingebracht. Die Locksubstanz, CD₃CN, befindet sich im Glas-NMR Röhrchen also zwischen Glas und FEP-Inliner und wird im Folgenden mit Filmmessung oder Lösemittel-Film gekennzeichnet. Die Messungen erfolgen auf einem 400 MHz Bruker Avance III Spektrometer mit einem 9.3980 T Kryomagneten und einem 5 mm-BBFO Probenkopf. ¹H-NMR-Spektren werden im ¹H/¹⁹F Kanal bei 400.17 MHz gemessen. ¹⁹F- und ³¹P-NMR-Spektren werden im Breitbandkanal bei 376.54 und 161.99 MHz gemessen. Die ¹H-NMR chemischen Verschiebungen werden auf Tetramethylsilan (TMS) bezogen und betragen für die Protonenrestsignale der Lösemittel D₂O (4.81 ppm), CDCl₃ (7.24 ppm) und CD₃CN (1.96 ppm). Die ¹⁹F chemischen Verschiebungen werden auf CFCl₃ bezogen und betragen für die internen Standards C₆F₆ (-162.9 ppm) oder C₆H₅CF₃ (-63.9 ppm). Die³¹P chemischen Verschiebungen werden auf H₃PO₄ (85 %) bezogen.

### Beispiele:

| | |
|---|---|
| **Methode A:** | 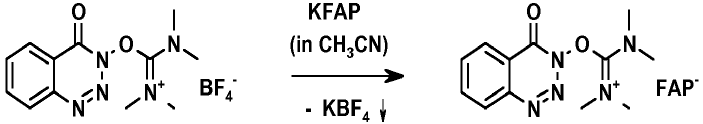 |
| **Methode B:** | 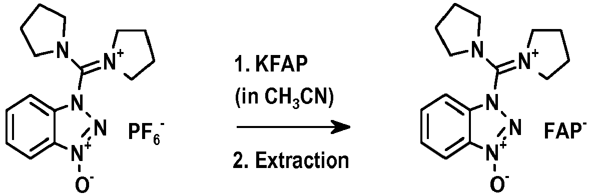 |

### Beispiel A) Synthese der Amidknüpfungsreagenzien:

### Methode A: Synthese aus Tetrafluorboraten

Eine Lösung von Kalium-tris(pentafluorethyl)trifluorophosphat, K[(C₂F₅)₃PF₃] (4.188 g, 8.65 mmol) in Acetonitril (15 mL) wird zu einer Lösung des Amidknüpfungsreagenzes enthaltend Tetrafluorboratanionen (8.65 mmol) in Acetonitril (15 mL) hinzugegeben und das Reaktionsgemisch bei 0 °C im Eis-Bad zwei Stunden gerührt. Das entstehende Gemisch wird filtriert. Der Rückstand, in der Hauptsache unlösliches Kaliumtetrafluoroborat, wird dreimal mit 10 mL von kaltem Acetonitril gewaschen und die Waschlösungen werden mit der Mutterlauge vereinigt. Von den vereinigten Lösungen wird das Acetonitril im Vakuum entfernt und der Rückstand (FAP-haltiges Peptidknüpfungsreagenz) im Vakuum (10⁻³ mbar) getrocknet und anschließend umkristallisiert.

### Methode B: Synthese aus Hexafluorophosphaten

Das Hexafluorophosphat-Peptidknüpfungsreagenz (4.74 mmol) und Kalium-tris(pentafluorethyl)trifluorophosphat, K[(C₂F₅)₃PF₃] (2.293 g, 4.74 mmol) werden in 20 mL Acetonitril suspendiert und bei Raumtemperatur gerührt bis eine klare Lösung entsteht. Das Acetonitril wird im Vakuum entfernt und der Rückstand im Vakuum (10⁻³ mbar) zwei Stunden getrocknet. Das Produkt wird aus dem Rückstand mit dem in Tabelle 1 angegebenen Lösemittel bzw. Lösemittelgemisch extrahiert und anschließend umkristallisiert.

**Tabelle 1: Übersicht zu den Synthese-Methoden, gekennzeichnet mit A oder B, für die neuen Amidknüpfungsreagenzien**

| Produkt | Edukt | Methode | Lösemittel bzw. Gemisch für Extraktion; Menge in mL/mmol | Lösemittel für die Umkristallisation | Ausbeute |
|---|---|---|---|---|---|
| DBTU-FAP | TDBTU | A | - | Dichlormethan / n-Hexan | 90% |
| | | | - | | |
| STU-FAP | TSTU | A | - | - | 86% |
| | | | | | |
| PfTU-FAP | PfTU | B | Dichlormethan; 6 mL/mmol | n-Hexan | 97% |
| OTT-FAP | HOTT | B | Dichlormethan; 3 mL/mmol | Diethylether | 82% |
| BTU-FAP | HBTU | B | Dichlormethan mit 2 % Acetonitril; 21 mL/mmol | - | 99% |
| BPyU-FAP | HBPyU | B | Dichlormethan; 20 mL/mmol | - | 93% |
| ATU-FAP | HATU | B | Dichlormethan mit 2.5 % Acetonitril; 60 mL/mmol | Diethylether | 52% |
| BOP-FAP | BOP | B | Dichlormethan; 6 mL/mmol | n-Hexan | 93% |
| PyBOP-FAP | PyBOP | B | Dichlormethan; 4 mL/mmol | n-Hexan | 92% |
| PyAOP-FAP | PyAOP | B | Dichlormethan; 4.3 mL/mmol | n-Hexan | 100% |
| TFF-FAP | TFFH | B | Dichlormethan; 4.0 mL/mmol | n-Hexan | 99% |
| COMU-FAP | COMU | B | Dichlormethan; 4.6 mL/mmol | n-Hexan | 97% |

### Charakterisierung der Amidknüpfungsreagenzien:

### O-(3,4-Dihydro-4-oxo-1,2,3-benzotriazin-3-yl)-N,N,N'N'-tetramethyluronium- tris(pentafluorethyl)trifluorophosphat (DBTU-FAP)

Schmelzpunkt: 124 °C.
Elementaranalyse:
experimentell, %: N 9.71, C 30.32, H 2.15;
berechnet für C₁₈H₁₆F₁₈N₅O₂P, %: N 9.90, C 30.56, H 2.28.
NMR (25 °C, Lösemittel: CD₃CN; δ in ppm):
¹H-NMR: 8.39 (d, d, ³*J*_{H,H} = 8.0 Hz, ⁴*J*_{H,H} = 1.4 Hz, 1H), 8.36 (d, m, ³*J*_{H,H} = 8.4 Hz, 1H), 8.21 (t, d, ³*J*_{H,H} = 7.6 Hz, ⁴*J*_{H,H} = 1.6 Hz, 1H), 8.04 (t, d, ³*J*_{H,H} = 7.6 Hz, ⁴*J*_{H,H} = 1.2 Hz, 1H), 3.23 (s, 12 H);
¹³C{¹H}-NMR: 162.89, 152.34, 144.99, 137.84, 135.23, 130.49, 126.76, 123.16, 41.41:
   ¹⁹F-NMR: -44.80 (d, m, ¹*J*_{F,P} = 890 Hz, 1F, PF), -80.89 (m, 3F, CF₃), -82.58 (m, 6F, 2CF₃),-88.21 (d, m, ¹*J*_{F,P} = 902 Hz, 2F, PF), -116.27 (br d, ²*J*_{F,P} = 82.9 Hz, 2F, CF₂), -116.89 (d, m, ²*J*_{F,P} = 97.7 Hz, 4F, 2CF₂):
   ³¹P-NMR : -147.91 (d, t, t, quin, ¹*J*_{P,F} = 902 Hz, ¹*J*_{P,F} = 889.2 Hz, ²*J*_{P,F} = 97.4 Hz, ²*J*_{P,F} = 83.8 Hz, 1P).
   Kristalldaten: (T = -150 °C): monoklin, a = 10.1170(5) Ǻ, b = 15.3535(12) Ǻ, c = 16.7415(10) Ǻ, β = 91.332(6).

### O-(N-Succinimidyl)-N,N,N',N'-tetramethyluroniumtris(pentafluorethyl)trifluorophosphat (STU-FAP)

Schmelzpunkt: 143 °C.
Elementaranalyse:
experimentell, %: N 6.19, C 27.19, H 2.41;
berechnet für C₁₅H₁₆F₁₈N₃O₃P, %: N 6.40, C 27.33. H 2.45.
NMR (25 °C, Lösemittel: CD₃CN; δ in ppm):
¹H-NMR: 3.13 (s, 12H), 2.84 (s, 4H);
¹³C{¹H} NMR : 170.90, 163.19, 41.18, 26.59;
¹⁹F NMR: -44.78 (d, m, ¹*J*_{F,P} = 890.6 Hz, 1F, PF), -80.86 (m, 3F, CF₃), -82.55 (m, 6F, 2CF₃), -88.20 (d, m, ¹*J*_{F,P} = 902.2 Hz, 2F, PF), -116.24 (br d, ²*J*_{F,P} = 83.0 Hz, 2F, CF₂), -116.85 (d, m, ²*J*_{F,P} = 97.8 Hz, 4F, 2CF₂);
³¹P NMR: -147.89 (d, t, t, quin, ¹*J*_{P,F} = 902.3 Hz, ¹*J*_{P,F} = 889.5 Hz, ²*J*_{P,F} = 97.7 Hz, ²*J*_{P,F} = 82.9 Hz, 1P).

### O-Pentafluorphenyl-N,N,N',N'-tetramethyluronium-tris(pentafluorethyl)trifluorophosphat (PfTU-FAP)

Schmelzpunkt: 49 °C.
Elementaranalyse:
experimentell, %: N 3.87, C 28.05, H 1.51;
berechnet für C₁₇H₁₂F₂₃N₂OP, %: N 3.85, C 28.04, H 1.66.
NMR (25 °C, Lösemittel: CD₃CN; δ in ppm):
¹H NMR: 3.12 (s, 12H);
¹³C{¹H}: 161.64, 41.24;
¹⁹F NMR: -44.83 (d, m, ¹*J*_{F,P} = 889.6 Hz, 1F, PF), -80.93 (m, 3F, CF₃), -82.62 (m, 6F, 2CF₃), -88.23 (d, m, ¹*J*_{F,P} = 902.3 Hz, 2F, PF), -116.30 (br d, ²*J*_{F,P} = 83.2 Hz, 2F, CF₂), -116.92 (d, m, ²*J*_{F,P} = 97.7 Hz, 4F, 2CF₂), -157.94 (d, ³*J*_{F,F} = 18.2 Hz, 2F, CF), -159.02 (t, ³*J*_{F,F} = 21.0 Hz, 1F, CF), -161.55 (d, t, ³*J*_{F,F} = 10.4 Hz, ³*J*_{F,F} = 10.4 Hz, 2F, CF);
³¹P NMR: -147.89 (d, t, t, quin, ¹*J*_{P,F} = 902.5 Hz, ¹*J*_{P,F} = 889.4 Hz, ²*J*_{P,F} = 97.6 Hz, ²*J*_{P,F} = 83.1 Hz, 1P).

### S-(1-Oxido-2-pyridyl)-N,N,N',N'-tetramethylthiouronium-tris(pentafluorethyl)trifluorophosphat (OTT-FAP)

Schmelzpunkt: 84 °C.
Elementaranalyse:
experimentell, %: N 6.51, C 28.79, H 2.29, S 4.69;
berechnet für C₁₆H₁₅F₁₈N₃OPS, %: N 6.27, C 28.67, H 2.26, S 4.78.
NMR (25 °C, Lösemittel: CD₃CN; δ in ppm):
¹H NMR: 8.32 (d, d, ³*J*_{H,H} = 6.4 Hz, ⁴*J*_{H,H} = 1.2 Hz, 1H), 7.73 (d, d, ³*J*_{H,H} = 7.7 Hz, ⁴*J*_{H,H} = 2.2 Hz, 1H), 7.47 (t, d, ³*J*_{H,H} = 5.9 Hz, ⁴*J*_{H,H} = 2.3 Hz), 7.42 (t, d, ³*J*_{H,H} = 7.7 Hz, ⁴*J*_{H,H} = 1.3 Hz, 1H), 3.20 (s, 12H);
¹³C{¹H} NMR: 172.21, 141.27, 131.56, 128.40, 127.53, 44.45;
¹⁹F NMR (CD₃CN, 25 °C): -44.75 (d, m, ¹*J*_{F,P} = 889.8 Hz, 1F, PF), -80.83 (m, 3F, CF₃), -82.52 (m, 6F, 2CF₃), -88.16 (d, m, ¹*J*_{F,P} = 902.3 Hz, 2F, PF), - 116.21 (br d, ²*J*_{F,P} = 83.1 Hz, 2F, CF₂), -116.81 (d, m, ²*J*_{F,P} = 97.7 Hz, 4F, 2CF₂);
³¹P NMR: -147.87 (d, t, t, quin, ¹*J*_{P,F} = 902.4 Hz, ¹*J*_{P,F} = 889.6 Hz, ²*J*_{P,F} = 97.7 Hz, ²*J*_{P,F} = 82.8 Hz, 1P).

### 1-(Dimethylamino(dimethylamonium-1-yliden)methyl)-1H-benzo[d][1,2,3]triazol-3-oxid-tris(pentafluorethyl)trifluorophosphat (BTU-FAP)

Schmelzpunkt: 135 °C.
Elementaranalyse:
experimentell, %: N 10.03, C 30.00; H 2.23;
berechnet für C₁₇H₁₆F₁₈N₅OP, %: N 10.31, C 30.06, H 2.37.
NMR (25 °C, Lösemittel: CD₃CN; δ in ppm):
¹H NMR: 8.05 (d, t, ³*J*_{H,H} = 8.4 Hz, ⁴*J*_{H,H} = 0.9 Hz, 1H), 7.94 (t, t, ³*J*_{H,H} = 7.4 Hz, *J* = 1.0 Hz, 1H), 7.71 (d, d, d, ³*J*_{H,H} = 8.1 Hz, ³*J*_{H,H} = 7.3 Hz, ⁴*J*_{H,H} = 0.5 Hz, 1H), 7.64 (d, m, ³*J*_{H,H} = 8.5 Hz, 1H), 3.38 (s, 6H), 3.03 (s, 6H);
¹³C{¹H1 NMR: 152.14, 134.44, 134.40, 128.43, 117.06, 115.01, 42.96, 42.64. ¹⁹F NMR: -44.78 (d, m, ¹*J*_{F,P} = 890.3 Hz, 1F, PF), -80.87 (m, 3F, CF₃), -82.57 (br m, 6F, 2CF₃), -88.19 (d, m, ¹*J*_{F,P} = 902.2 Hz, 2F, PF), -116.26 (br d, ²*J*_{F,P} = 83.9 Hz, 2F, CF₂), -116.85 (d, m, ²*J*_{F,P} = 98.3 Hz, 4F, 2CF₂);
³¹P NMR: -147.88 (d, t, t, quin, ¹*J*_{P,F} = 902.1 Hz, ¹*J*_{P,F} = 889.0 Hz, ²*J*_{P,F} = 96.6 Hz, ²*J*_{P,F} = 83.8 Hz, 1P).

### 1-(Pyrrolidino(pyrrolidinium-1-yliden)methyl)-1H-benzo[d][1,2,3]triazol-3-oxid- tris(pentafluorethyl)trifluorophosphat (BPyU-FAP)

Schmelzpunkt: 173 °C (Zersetzung.).

Elementaranalyse:
experimentell, %: N 9.58, C 34.76, H 2.98;
berechnet für C₂₁H₂₀F₁₈N₅OP, %: N 9.58, C 34.49, H 2.78.
NMR (25 °C, Lösemittel: CD₃CN; δ in ppm):
¹H NMR: 8.06 (d, ³*J*_{H,H} = 8.4 Hz, 1H), 7,94 (d, d, d, ³*J*_{H,H} = 8.4 Hz, ³*J*_{H,H} = 7.2 Hz, ⁴*J*_{H,H} = 1.0 Hz, 1H), 7.70 (br t, ³*J*_{H,H} = 7.7 Hz, 1H), 7.66 (d, ³*J*_{H,H} = 8.5, 1H), 3.93 (br m, 4H), 3.67 (very br m, 2H), 3.46 (very br m, 2H), 2.17 (very br m, 4H), 1.99 (very br m, 4H);
¹³C{¹H} NMR: 146.27, 134.61, 133.81, 133.44, 128.01, 117.08, 114.35, 54.04 (br), 52.75 (br), 26.91 (br), 25.17 (br);
¹⁹F NMR: -44.81 (d, m, ¹*J*_{F,P} = 889.4 Hz, 1F, PF), -80.90 (m, 3F, CF₃), -82.60 (m, 6F, 2CF₃), -88.21 (d, m, ¹*J*_{F,P} = 902.3 Hz, 2F, PF), -116.29 (br d, ²*J*_{F,P} = 82.0 Hz, 2F, CF₂), -116.90 (dm, ²*J*_{F,P} = 97.8 Hz, *J* = 8.1 Hz, 4F, 2CF₂);
³¹P NMR: -147.92 (d, t, t, quin, ¹*J*_{P,F} = 903.3 Hz, ¹*J*_{P,F} = 889.5 Hz, ²*J*_{P,F} = 97.5 Hz, ²*J*_{P,F} = 83.2 Hz, 1P).
Kristalldaten: (T = -150 °C): monoklin, a = 8.8373(5) Ǻ, b = 31.0766(19) Ǻ, c = 10.4906(6) Ǻ, β = 110.133(6).

### 1-(Dimethylamino(dimethylamonium-1-yliden)methyl)-1H-azabenzo[d][1,2,3]triazol-3-oxid-tris(pentafluorethyl)trifluorophosphat (ATU-FAP)

Schmelzpunkt: >140 °C (Zersetzung).

Elementaranalyse:
experimentell, %: N 12.27, C 28.29, H 2.02;
berechnet für C₁₆H₁₅F₁₈N₆OP, %: N 12.35, C 28.25, H 2.22.
NMR (25 °C, Lösemittel: CD₃CN; δ in ppm):
¹H NMR: 8.84 (d, d, ³*J*_{H,H} = 4.5 Hz, ⁴*J*_{H,H} = 1.1 Hz, 1H), 8.12 (d, d, ³*J*_{H,H} = 8.6 Hz, ⁴*J*_{H,H} = 1.3 Hz, 1H), 7.93 (d, d, ³*J*_{H,H} = 8.6 Hz, ⁴*J*_{H,H} = 4.5 Hz, 1H), 3.37 (s, 6H), 3.04 (s, 6H);
¹³C{¹H} NMR: 152.19, 150.87, 144.97, 128.85, 128.29, 125.00, 43.09, 42.69; ¹⁹F NMR: -44.80 (d, m, ¹*J*_{F,P} = 889.5 Hz, 1F, PF), -80.90 (m, 3F, CF₃), -82.59 (br m, 6F, 2CF₃), -88.21 (d, m, ¹*J*_{F,P} = 902.2 Hz, 2F, PF), -116.28 (br d, ²*J*_{F,P} = 83.0 Hz, 2F, CF₂), -116.89 (d, m, ²*J*_{F,P} = 97.7 Hz, 4F, 2CF₂);
³¹P NMR: -147.88 (d, t, t, quin, ¹*J*_{P,F} = 902.6 Hz, ¹*J*_{P,F} = 889.2 Hz, ²*J*_{P,F} = 98.8 Hz, ²*J*_{P,F} = 83.5 Hz, 1P).

### (Benzotriazol-1-yloxy)tris(dimethylamino)phosphonium-tris(pentafluorethyl)trifluorophosphat (BOP-FAP)

Schmelzpunkt: 91 °C.

Elementaranalyse:
experimentell, %: N 10.99, C 29.14, H 3.11;
berechnet für C₁₈H₂₂F₁₈N₆OP₂, %: N 11.32, C 29.12, H 2.99.
NMR (25 °C, Lösemittel: CD₃CN; δ in ppm):
¹H NMR: 8.16 (d, t, ³*J*_{H,H} = 8.5 Hz, ⁴*J*_{H,H} = 0.9 Hz, 1H), 7.85 - 7.75 (m 2H), 7,62 (d, d, d, ³*J*_{H,H} = 8.4 Hz, ³*J*_{H,H} = 6.5 Hz, ⁴*J*_{H,H} = 1.5 Hz, 1H), 2.84 (d, ³*J*_{H,P} = 10.6, 18H);
¹³C{¹H} NMR: 144.14, 131.58, 129.01, 127.25, 121.71, 109.27, 38.96 (d, ²*J*_{C,P} = 4.3 Hz, CH₃).
¹⁹F NMR: -44.79 (d, m, ¹*J*_{F,P} = 889.5 Hz, 1F, PF), -80.88 (m, 3F, CF₃), -82.57 (m, 6F, 2CF₃), -88.20 (d, m, ¹*J*_{F,P} = 898.5 Hz, 2F, PF), -116.26 (br d, ²*J*_{F,P} = 82.8 Hz, 2F, CF₂), -116.87 (d, m, ²*J*_{F,P} = 97.7 Hz, 4F, 2CF₂);
³¹P NMR: 43.67 (m, 1P), -147.91 (d, t, t, quin, ¹*J*_{P,F} = 902.7 Hz, ¹*J*_{P,F} = 889.2 Hz, ²*J*_{P,F} = 97.5 Hz, ²*J*_{P,F} = 83.7 Hz, 1P).

### (Benzotriazol-1-yloxy)tris(pyrrolidino)phosphonium-tris(pentafluorethyl)trifluorophosphat (PyBOP-FAP)

Schmelzpunkt: 66 °C.

Elementaranalyse:
experimentell, %: N 9.72, C 34.55, H 3.30;
berechnet für C₂₄H₂₈F₁₈N₆OP₂, %: N 10.24, C 35.13, H 3.44.
NMR (25 °C, Lösemittel: CD₃CN; δ in ppm):
¹H NMR: 8.12 (d, t, ³*J*_{H,H} = 8.5 Hz, ⁴*J*_{H,H} = 0.8 Hz, 1H), 7.80 - 7.70 (m 2H), 7,59 (d, d, d, ³*J*_{H,H} = 8.4 Hz, ³*J*_{H,H} = 5.6 Hz, ⁴*J*_{H,H} = 2.4 Hz, 1H), 3.34 (m, 12H), 1.88 (m, 12H);
¹³C{¹H} NMR: 144.07 (d, ⁴*J*_{C,P} = 0.4 Hz), 131.29, 128.92 (d, ³*J*_{C,P} = 0.9 Hz), 127.16, 121.57, 109.38, 49.24 (d, ²*J*_{C,P} = 4.9 Hz), 26.80 (d, ³*J*_{C,P} = 9.2 Hz); ¹⁹F NMR: -44.81 (d, m, ¹*J*_{F,P} = 889.6 Hz, 1F, PF), -80.90 (m, 3F, CF₃), -82.60 (m, 6F, 2CF₃), -88.22 (d, m, ¹*J*_{F,P} = 902.2 Hz, 2F, PF), -116.29 (br d, ²*J*_{F,P} = 83.1 Hz, 2F, CF₂), -116.90 (d, m, ²*J*_{F,P} = 97.8 Hz, 4F, 2CF₂);
³¹P NMR: 31.01 (br s, 1P), -147.89 (d, t, t, quin, ¹*J*_{P,F} = 902.6 Hz, ¹*J*_{P,F} = 889.7 Hz, ²*J*_{P,F} = 97.0 Hz, ²*J*_{P,F}= 83.7 Hz, 1P).

### (7-Azabenzotriazol-1-yloxy)tris(pyrrolidino)phosphonium-tris(pentafluorethyl)trifluorophosphat (PyAOP-FAP)

Schmelzpunkt: 77 °C.

Elementaranalyse: experimentell, %: N 11.77, C 33.68, H 3.39;
berechnet für C₂₃H₂₇F₁₈N₇OP₂, %: N 11.94, C 33.63, H 3.31.
NMR (25 °C, Lösemittel: CD₃CN; δ in ppm):
¹H NMR: 8.84 (d, ³*J*_{H,H} = 4.5 Hz, 1H), 8.54 (d, ³*J*_{H,H} = 8.4 Hz, 1H), 7,62 (d, d, ³*J*_{H,H} = 8.4 Hz, ³*J*_{H,H} = 5.6 Hz, 1H), 3.37 (m, 12H), 1.88 (m, 12H);
¹³C{¹H} NMR: 154.25, 141.05, 135.92, 131.17, 123.32, 49.26 (d, ²*J*_{C,P} = 4.5 Hz), 26.84 (d, ³*J*_{C,P} = 9.3 Hz);
¹⁹F NMR: -44.78 (d, m, ¹*J*_{F,P} = 889.7 Hz, 1F, PF), -80.85 (m, 3F, CF₃), -82.54 (m, 6F, 2CF₃), -88.17 (d, m, ¹*J*_{F,P} = 902.4 Hz, 2F, PF), -116.24 (br d, ²*J*_{F,P} = 82.7 Hz, 2F, CF₂), -116.85 (d, m, ²*J*_{F,P} = 97.7 Hz, 4F, 2CF₂);
³¹P NMR: 30.93 (br s, 1P), -147.90 (d, t, t, quin, ¹*J*_{P,F} = 902.4 Hz, 1*J*_{P,F} = 889.6 Hz, ²*J*_{P,F} = 97.7 Hz, ²*J*_{P,F} = 83.1 Hz, 1P).

### Fluor-N,N,N',N'-tetramethylformamidinium-tris(pentafluorethyl)trifluorophosphat (TFF-FAP)

Schmelzpunkt: 82 °C.

Elementaranalyse: experimentell, %: N 4.92, C 23.48, H 2.14;
berechnet für C₁₁H₁₂F₁₉N₂P, %: N 4.97, C 23.42, H 2.14.

NMR (25 °C, Lösemittel: CD₃CN; δ in ppm):
¹H NMR: 3.16 (d, ⁴*J*_{H,F} = 3.0 Hz, 12H);
¹³C{¹H} NMR: 120.13 (m, ¹*J*_{C,F} = 285.2 Hz), 49.26 (d, ²*J*_{C,P} = 4.5 Hz), 26.84 (d, ³*J*_{C,P} = 9.3 Hz), 39.11;
¹⁹F NMR: -44.77 (d, m, ¹*J*_{F,P} = 889.5 Hz, 1F, PF), -45.17 (tridezett, ⁴*J*_{F,H} = 3.0 Hz, 1F, CF), -80.92 (m, 3F, CF₃), -82.61 (m, 6F, 2CF₃), -88.25 (d, m, ¹*J*_{F,P} = 904.2 Hz, 2F, PF), -116.34 (br d, ²*J*_{F,P} = 82.3 Hz, 2F, CF₂), -116.92 (d, m, ²*J*_{F,P} = 97.7 Hz, 4F, CF₂);
³¹P NMR: -147.90 (d, t, t, quin, ¹*J*_{P,F} = 904.2 Hz, ¹*J*_{P,F} = 889.5 Hz, ²*J*_{P,F} = 97.7 Hz, ²*J*_{P,F} = 82.3 Hz, 1P).

### (1-Cyano-2-ethoxy-2-oxoethylidenaminooxy)-dimethylamino-morpholino-carbenium-tris(pentafluorethyl)trifluorophosphat (COMU-FAP)

Schmelzpunkt: 79 °C.
Elementaranalyse: experimentell, %: N 7.69, C 29.63, H 2.44;
berechnet für C₁₈H₁₉F₁₈N₄O₄P, %: N 7.69, C 29.68, H 2.63.
NMR (25 °C, Lösemittel: CD₃CN; δ in ppm):
¹H NMR: 4.49 (q, ³*J*_{H,H} = 7.1 Hz, 2H, CH₂), 3.82 (t, ³*J*_{H,H} = 4.7 Hz, 4H, CH₂), 3.56 (t, ³*J*_{H,H} = 4.7 Hz, 4H, CH₂), 3,19 (s, 6H, CH₃), 1.40 (t, ³*J*_{H,H} = 7.1 Hz, 2H, CH₃);
¹³C{¹H} NMR: 159.82, 155.47, 134.43, 106.05, 65.36, 64.65, 49.36, 40.25, 12.84;
¹⁹F NMR: -44.83 (d, m, ¹*J*_{F,P} = 889.2 Hz, 1F, PF), -80.86 (m, 3F, CF₃), -82.56 (m, 6F, 2CF₃), -88.26 (d, m, ¹*J*_{F,P} = 901.8 Hz, 2F, PF), -116.24 (br d, ²*J*_{F,P} = 83.1 Hz, 2F, CF₂), -116.87 (d, m, ²*J*_{F,P} = 98.2 Hz, 4F, 2CF₂);
³¹P NMR: -147.92 (d, t, t, quin, ¹*J*_{P,F} = 901.8 Hz, ¹*J*_{P,F} = 889.6 Hz, ²*J*_{P,F} = 98.2 Hz, ²*J*_{P,F} = 83.1 Hz, 1P).

### Beispiel B) Löslichkeit der Amidknüpfungsreagenzien

Alle Löslichkeitsbestimmungen werden in einer tarierten gasdichten 4 mL Flasche mit Magnetrührkern vorgenommen. Das Amidknüpfungsreagenz (mindestens 5 mg) wird eingewogen und eine bestimmte Menge des zu untersuchenden Lösemittels wird zugegeben. Das Gemisch wird 2-5 Minuten gerührt. Fals noch ungelöster Feststoff vorliegt, wird zusätzlich Lösungsmittel (50-100 µL) zugegeben und das Gemisch wird wieder gerührt. Sobald eine klare Lösung vorliegt, wird das Experiment beendet. Die nachfolgende Tabelle 2 fasst die Löslichkeiten der untersuchten Peptidknüpfungsreagenzien zusammen.

**Tabelle 2: Löslichkeiten der Amidknüpfungsreagenzien in mg/mL; FAP⁻ bedeutet in Tabelle 2 [(C₂F₅)₃PF₃]⁻**

| Reagenz | CH₂Cl₂ | CH₃CN | 9 CH₂Cl₂ : 1 CH₃CN | THF | DMF |
|---|---|---|---|---|---|
| HDBTU | 3.75 | 240 | 17 | <3.3 | 250 |
| **DBTU-FAP** | **>780** | **>860** | **480** | **>780** | **>1125** |
| TSTU | < 1.2 | 265 | 2.9 | < 1.2 | 220 |
| **STU-FAP** | **3.0** | **> 2000** | **110** | **690** | **> 2000** |
| PfTU | < 1.2 | 1000 | 2.5 | < 1.2 | 1000 |
| **PfTU-FAP** | **1000** | **> 2000** | **> 2000** | **1000** | **> 2000** |
| HOTT | 59 | 810 | 180 | < 1.2 | 690 |
| **OTT-FAP** | **690** | **> 2000** | **> 2000** | **1400** | **> 2000** |
| HBTU | <1.1 | 124 | <2.8 | <3.6 | 200 |
| **BTU-FAP** | **6.9** | **>1040** | **120** | **364** | **>1200** |
| HBPyU | 68.3 | 510 | 118 | <3.3 | 540 |
| **BPyU-FAP** | **57** | **>1020** | **190** | **510** | **>1100** |
| HATU | < 1.2 | 212 | 1.2 | < 1.2 | 370 |
| **ATU-FAP** | **< 1.2** | **> 2000** | **67** | **670** | **> 2000** |
| BOP | >380 | 510 | >335 | <4.2 | 660 |
| **BOP-FAP** | **>1040** | **>700** | **>800** | **>1200** | **>1100** |
| PyBOP | 680 | 830 | 660 | 4.3 | 670 |
| **PyBOP-FAP** | **> 1650** | **>1550** | **> 1500** | **>1500** | **> 1550** |
| PyAOP | 1000 | 1000 | 670 | 6.0 | 670 |
| **PyAOP-FAP** | **> 2000** | **> 2000** | **> 2000** | **> 2000** | **> 2000** |
| TFFH | 220 | 1360 | 595 | 6.3 | 610 |
| **TFF-FAP** | **1190** | **4490** | **> 2000** | **880** | **> 2000** |
| COMU | 82 | 565 | 294 | 4.1 | 530 |
| **COMU-FAP** | **180** | **905** | **> 2000** | **349** | **> 1500** |

**Tabelle 2 - Fortsetzung:**

| Reagenz | Hexan | Toluol |
|---|---|---|
| HDBTU | < 1.2 | < 1.2 |
| **DBTU-FAP** | **< 1.2** | **< 1.2** |
| TSTU | < 1.2 | < 1.2 |
| **STU-FAP** | **< 1.2** | **< 1.2** |
| PfTU | < 1.2 | < 1.2 |
| **PfTU-FAP** | **< 1.2** | **< 1.2** |
| HOTT | < 1.2 | < 1.2 |
| **OTT-FAP** | **< 1.2** | **< 1.2** |
| HBTU | < 1.2 | < 1.2 |
| **BTU-FAP** | **< 1.2** | **< 1.2** |
| HBPyU | < 1.2 | < 1.2 |
| **BPyU-FAP** | **< 1.2** | **< 1.2** |
| HATU | < 1.2 | < 1.2 |
| **ATU-FAP** | **< 1.2** | **< 1.2** |
| BOP | < 1.2 | < 1.2 |
| **BOP-FAP** | **< 1.2** | **< 1.2** |
| PyBOP | < 1.2 | < 1.2 |
| **PyBOP-F AP** | **< 1.2** | **5.0** |
| PyAOP | < 1.2 | < 1.2 |
| **PyAOP-FAP** | **< 1.2** | **b** |
| TFFH | 2.2 | 4.8 |
| **TFF-FAP** | **6.2** | **13.1** |
| COMU | < 1.2 | 3.2 |
| **COMU-FAP** | **2.1** | **4.5** |

| | | |
|---|---|---|
| **b** bedeutet, dass die Bildung von zwei flüssigen Phasen beobachtet wurde | | |

Die Löslichkeit in den unpolaren Lösemitteln Hexan und Toluol ist wie erwartet kaum verändert, da es sich nach wie vor um ionische Verbindungen handelt. Man kann jedoch die Tendenz der Löslichkeitsverbesserung bei Vergleich von TFFH und TFF-FAP und COMU und COMU-FAP erkennen.

### Beispiel C) Aktivierungsexperimente mit Z-Aib-OH

Z-Aib-OH (N-Benzyloxycarbonyl-*α*-amino-*iso*-buttersäure) wurde für die Aktivierungsexperimente ausgewählt, weil sie eine Carboxylgruppe trägt, die nicht leicht zu aktivieren ist, wie zuvor erwähnt. In einem typischen Experiment werden zu einer 0.1 M Lösung von Z-Aib-OH in THF mit einem Äquivalent Amidknüpfungsreagenz zwei Äquivalente TMP zugegeben. In bestimmten Zeitintervallen (4, 20 und 40 Minuten) werden -20 µL des Reaktionsgemisches entnommen und in 0.6 - 0.7 mL deuteriertem Chloroform in einem NMR-Röhrchen gelöst. Die Proben werden bis zur NMR-spektroskopischen Untersuchung bei 0 °C gekühlt (für ca. 20-45 Minuten). Die Intensitäten der Benzylprotonen-NMR-Signale der Säure (5.05 ppm) und des aktiven Esters (-5.20 ppm) werden miteinander verglichen, um den Umsatz der Reaktion zu berechnen [L. A. Carpino, A. El-Faham, J. Org. Chem. 1994, 59, 695-698]. Alle Resultate werden in der Tabelle 3 zusammengestellt.

**Tabelle 3: Aktivierung von Z-Aib-OH durch die in der Tabelle aufgeführten Amidknüpfungsreagenzien**

| Reagenz | Zeit | Umsatz, % für X⁻ = BF₄⁻ | Umsatz, % für X⁻ = [(C₂F₅)₃PF₃]⁻ |
|---|---|---|---|
| 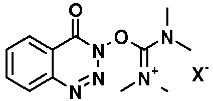 | 4 Minuten | 6 | 90 |
| | 20 Minuten | 36 | 95 |
| | 40 Minuten | 62 | 99 |
| 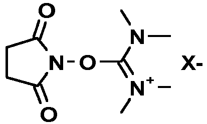 | 4 Minuten | 0 | 26 |
| | 20 Minuten | 0 | 50 |
| | 40 Minuten | 0 | 63 |

| Reagenz | Zeit | Umsatz, % für X⁻ = PF₆⁻ | Umsatz, % für X⁻ = [(C₂F₅)₃PF₃]⁻ |
|---|---|---|---|
| 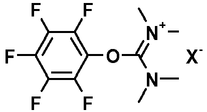 | 4 Minuten | 52 | 91 |
| | 20 Minuten | 80 | 95 |
| | 40 Minuten | 87 | 97 |
| 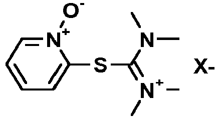 | 4 Minuten | 3.7 | 8 |
| | 20 Minuten | 3.7 | 15 |
| | 40 Minuten | 4.0 | 21 |

| Reagenz | Zeit | Umsatz, % für X⁻ = PF₆⁻ | Umsatz, % für X⁻ = [(C₂F₅)₃PF₃]⁻ |
|---|---|---|---|
| 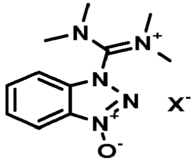 | 4 Minuten | 3 | 79 |
| | 20 Minuten | 2 | 89 |
| | 40 Minuten | 4 | 95 |
| 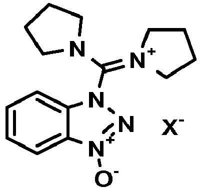 | 4 Minuten | 11 | 87 |
| | 20 Minuten | 21 | 97 |
| | 40 Minuten | 24 | 97 |
| 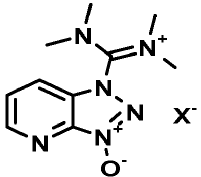 | 4 Minuten | 0 | 100 |
| | 20 Minuten | 0 | 100 |
| | 40 Minuten | 6 | 100 |
| 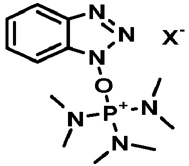 | 4 Minuten | 7 | 28 |
| | 20 Minuten | 22 | 64 |
| | 40 Minuten | 43 | 76 |
| 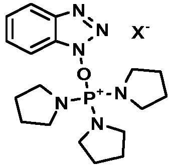 | 4 Minuten | 31 | 53 |
| | 20 Minuten | 65 | 83 |
| | 40 Minuten | 82 | 88 |
| 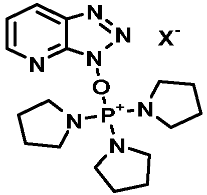 | 4 Minuten | 62 | 71 |
| | 20 Minuten | 89 | 90 |
| | 40 Minuten | 93 | 93 |

Mit den Amidknüpfungsreagenzien enthaltend das Anion [(C₂F₅)₃F₃P]⁻ läuft die Aktivierung der Säure und damit auch die weiteren Schritte der Amidbindungsbildung in den meisten Fällen schneller ab. Die Beschleunigung der Aktivierung ist für die Amidknüpfungsreagenzien PyBOP-FAP und PyAOP-FAP weniger ausgeprägt.

### Beispiel D) Synthese von Ac-Phe-Ala-OMe

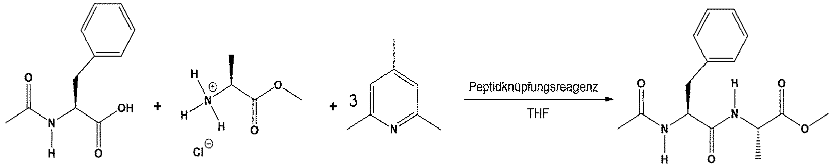

Bei dieser Reaktion kann nicht nur der Umsatz sondern auch der Epimerisierungsgrad mit Hilfe von ¹H NMR Spektroskopie leicht analysiert werden [B. Weinstein, A. E. Pritchard, J. Chem. Soc., Perkin Trans. 1 1972, 1015].

In einem typischen Experiment werden drei Äquivalente TMP (Collidin) zu einer Ac-Phe-OH-Lösung (Ac-Phe-OH = acetylgeschützes Phenylalanin) in THF (0.2 M) mit einem Äquivalent Ala-OMe Hydrochlorid (Ala-OMe = Alaninmethylester) und einem Äquivalent Peptidknüpfungsreagenz zugegeben. In bestimmten Zeitabständen (5, 10 und 15 Minuten) werden 40 µL des Gemisches in 0.6-0.7 mL Acetonitril-d₃ in einem NMR-Röhrchen gelöst und bei 0 °C gekühlt. Wenn alle drei Proben vorbereitet sind, werden ¹H NMR-Spektren von diesen Proben bei 25 °C gemessen. Dabei liegt das Dublett der Alanin-Methylgruppe im Dipeptid bei δ = 1.32 ppm und im Epimer bei δ = 1.25 ppm, das Dublett von nicht umgesetztem Ala-OMe Hydrochlorid im Bereich von δ = 1.40 - 1.50 ppm. Durch Integration von drei Signalen wird der Umsatz und der Epimerisierungsgrad des Dipeptids berechnet. Die Ergebnisse des Umsatzes sind in der Tabelle 4 und des Epimerisierungsgrads in der Tabelle 5 zusammengestellt.

**Tabelle 4:**

| Reagenz | Zeit | Umsatz, % für X⁻ = BF₄⁻ | Umsatz, % für X⁻ = [(C₂F₅)₃PF₃]⁻ |
|---|---|---|---|
| 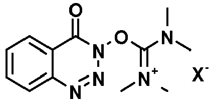 | 5 Minuten | 74 | 100 |
| | 10 Minuten | 89 | 100 |
| | 15 Minuten | 94 | 100 |
| 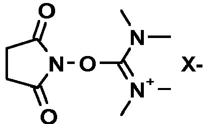 | 5 Minuten | 9 | 41 |
| | 10 Minuten | 11 | 55 |
| | 15 Minuten | 20 | 67 |

| Reagenz | Zeit | Umsatz, % für X⁻ = PF₆⁻ | Umsatz, % für X⁻ = [(C₂F₅)₃PF₃]⁻ |
|---|---|---|---|
| 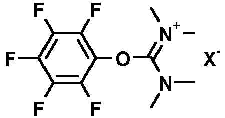 | 5 Minuten | 32 | 44 |
| | 10 Minuten | 55 | 63 |
| | 15 Minuten | 68 | 73 |
| 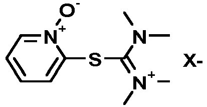 | 5 Minuten | 0 | 5 |
| | 10 Minuten | 1,7 | 13 |
| | 15 Minuten | 2 | 19 |
| 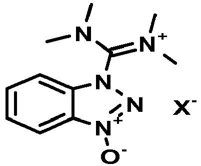 | 5 Minuten | 5,3 | 93 |
| | 10 Minuten | 6 | 100 |
| | 15 Minuten | 6,3 | 100 |
| 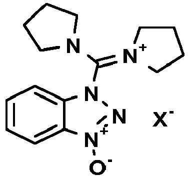 | 5 Minuten | 49 | 100 |
| | 10 Minuten | 69 | 100 |
| | 15 Minuten | 76 | 100 |
| 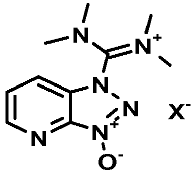 | 5 Minuten | 58 | 100 |
| | 10 Minuten | 69 | 100 |
| | 15 Minuten | 76 | 100 |

| Reagenz | Zeit | Umsatz, % für X⁻ = PF₆⁻ | Umsatz, % für X⁻ = [(C₂F₅)₃PF₃]⁻ |
|---|---|---|---|
| 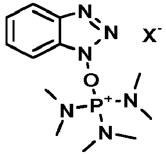 | 5 Minuten | 44 | 67 |
| | 10 Minuten | 59 | 77 |
| | 15 Minuten | 68 | 80 |
| 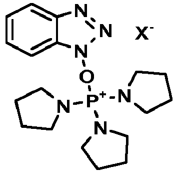 | 5 Minuten | 71 | 85 |
| | 10 Minuten | 83 | 91 |
| | 15 Minuten | 87 | 94 |
| 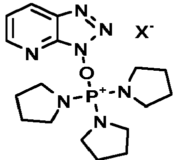 | 5 Minuten | 90 | 92 |
| | 10 Minuten | 97 | 100 |
| | 15 Minuten | 100 | 100 |

Die Umsätze werden aus den Integralen der Alanin-Methylgruppe von Ala-OMe, Dipeptid und Epimer berechnet.

Bei allen Amidknüpfungsreagenzien wird eine beschleunigte Reaktion beobachtet. Der Vorteil der neuen Peptidknüpfungsreaktionen ist insbesondere der Zeitgewinn bei der Peptidsynthese durch schnellere Reaktionszeiten.

Die Ergebnisse des Epimerisierungsgrades sind in der Tabelle 5 zusammengestellt.

**Tabelle 5:**

| Reagenz | Zeit | Epimerisierung, % für X⁻ = BF₄⁻ | Epimersierung, % für X⁻ = [(C₂F₅)₃PF₃]⁻ |
|---|---|---|---|
| 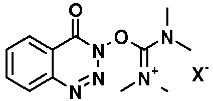 | 5 Minuten | 0 | 0 |
| | 10 Minuten | 0 | 0 |
| | 15 Minuten | 0,6 | 0,4 |
| 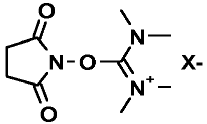 | 5 Minuten | b | 4,0 |
| | 10 Minuten | b | 4,0 |
| | 15 Minuten | 3,8 | 5,1 |

| Reagenz | Zeit | Epimerisierung, % für X⁻= PF₆- | Epimerisierung, % für X⁻ = [(C₂F₅)₃PF₃]⁻ |
|---|---|---|---|
| 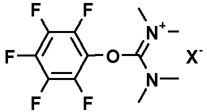 | 5 Minuten | 3,9 | 6,2 |
| | 10 Minuten | 6,0 | 9,0 |
| | 15 Minuten | 8,1 | 9,9 |
| 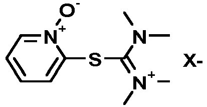 | 5 Minuten | b | b |
| | 10 Minuten | b | 9,1 |
| | 15 Minuten | b | 9,1 |
| 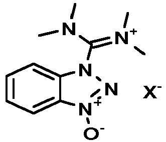 | 5 Minuten | b | 3,1 |
| | 10 Minuten | b | 2,8 |
| | 15 Minuten | b | 3,2 |
| 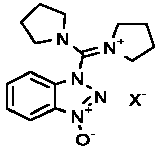 | 5 Minuten | 8,2 | 3,3 |
| | 10 Minuten | 7,3 | 1,8 |
| | 15 Minuten | 5,0 | 2,4 |
| 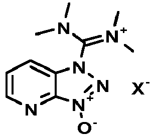 | 5 Minuten | 0 | 1,6 |
| | 10 Minuten | 1,0 | 1,2 |
| | 15 Minuten | 1,0 | 1,4 |
| 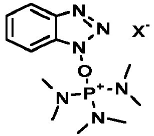 | 5 Minuten | 3,5 | 1,9 |
| | 10 Minuten | 3,9 | 3,0 |
| | 15 Minuten | 4,2 | 3,4 |
| 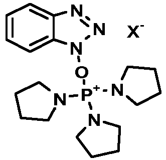 | 5 Minuten | 2,2 | 2,4 |
| | 10 Minuten | 2,8 | 2,3 |
| | 15 Minuten | 3,1 | 2,4 |
| 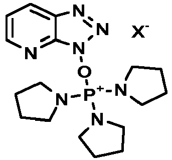 | 5 Minuten | 0,7 | 0,9 |
| | 10 Minuten | 0,7 | 0,6 |
| | 15 Minuten | 0,8 | 1,0 |

Wird in der Tabelle der Buchstabe b verwendet, so war es nicht möglich, den Epimerisierungsgrad auf Grund zu niedriger Intensität des Signals zu berechnen.

In einigen Fällen wird eine Erniedrigung des Epimerisierungsgrades festgestellt.

## Patentansprüche

1. Verfahren zur Herstellung einer Amidbindung durch Reaktion einer Säure mit einem primären oder sekundären Amin in Gegenwart einer Base mit Hilfe eines Amidknüpfungsreagenzes, **dadurch gekennzeichnet, dass** mindestens ein Amidknüpfungsreagenz enthaltend ein Anion der Formel (I) verwendet wird,
[P(CₙF₂ₙ₊₁)_{y}F_{6-y}]⁻ (I),
wobei
n bei jedem Auftreten unabhängig für 1, 2, 3, 4, 5, 6, 7 oder 8 steht und
y für 1, 2, 3 oder 4 steht.

2. Verfahren nach Anspruch 1, wobei Amidknüpfungsreagenzien mit Anionen der Formel (I) verwendet werden, in denen die Variable n bei jedem Auftreten unabhängig für 2, 3 oder 4 steht.

3. Verfahren nach Anspruch 1 oder 2, wobei Amidknüpfungsreagenzien mit Anionen der Formel (I) verwendet werden, die aus der Gruppe [(C₂F₅)₃PF₃]⁻, [(C₃F₇)₃PF₃]⁻, [(C₄F₉)₃PF₃]⁻, [(C₂F₅)₂PF₄]⁻, [(C₃F₇)₂PF₄]⁻, [(C₄F₉)₂PF₄]⁻, [(C₂F₅)PF₅]⁻, [(C₃F₇)PF₅]⁻ und [(C₄F₉)PF₅]⁻ ausgewählt werden.

4. Verfahren nach einem oder mehreren der Ansprüche 1 bis 3, **dadurch gekennzeichnet, dass** das Kation des Amidknüpfungsreagenzes ein Uronium-, ein Thiouronium-, ein Guanidinium-, ein Aminium-, ein Carbonium-, ein Imidazolium- oder ein Phosphonium-Kation bedeutet.

5. Verfahren nach einem oder mehreren der Ansprüche 1 bis 4, **dadurch gekennzeichnet, dass** eine Peptidbindung hergestellt wird.

6. Verbindungen aus der folgenden Gruppe von Verbindungen, wobei FAP⁻ für ein Anion der Formel (I) steht, wie in Anspruch 1 beschrieben:

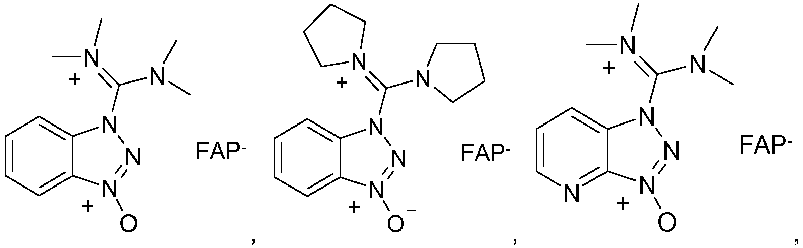

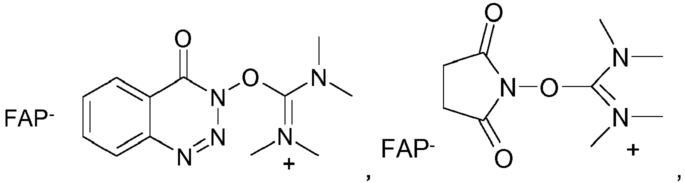

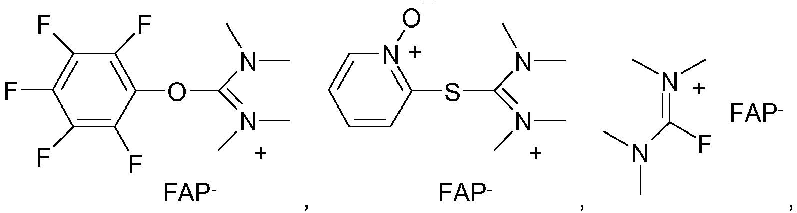

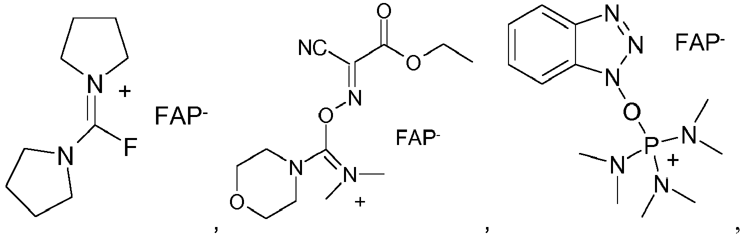

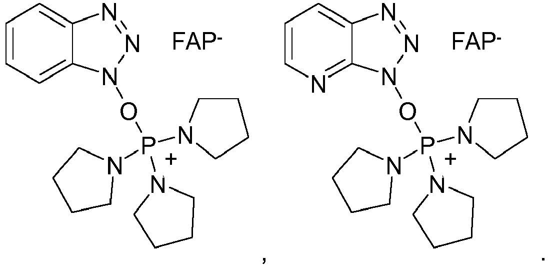

7. Verbindungen nach Anspruch 6, wobei FAP⁻ für [(C₂F₅)₃PF₃]⁻ oder [(*n*-C₄F₉)₃PF₃]⁻ steht.

## Claims

1. Process for the formation of an amide bond by reaction of an acid with a primary or secondary amine in the presence of a base with the aid of an amide linking reagent, **characterised in that** use is made of at least one amide linking reagent containing an anion of the formula (I),
[P(CₙF₂ₙ₊₁)_{y}F_{6-y}]⁻ (I),
where
n stands on each occurrence, independently, for 1, 2, 3, 4, 5, 6, 7 or 8 and
y stands for 1, 2, 3 or 4.

2. Process according to Claim 1, in which use is made of amide linking reagents containing anions of the formula (I) in which the variable n stands on each occurrence, independently, for 2, 3 or 4.

3. Process according to Claim 1 or 2, in which use is made of amide linking reagents containing anions of the formula (I) selected from the group [(C₂F₅)₃PF₃]⁻, [(C₃F₇)₃PF₃]⁻, [(C₄F₉)₃PF₃]⁻, [(C₂F₅)₂PF₄]⁻, [(C₃F₇)₂PF₄]⁻, [(C₄F₉)₂PF₄]⁻, [(C₂F₅)PF₅]⁻, [(C₃F₇)PF₅]⁻ and [(C₄F₉)PF₅]⁻.

4. Process according to one or more of Claims 1 to 3, **characterised in that** the cation of the amide linking reagent is a uronium, thiouronium, guanidinium, aminium, carbonium, imidazolium or phosphonium cation.

5. Process according to one or more of Claims 1 to 4, **characterised in that** a peptide bond is formed.

6. Compounds from the following group of compounds, where FAP⁻ stands for an anion of the formula (I), as described in Claim 1:

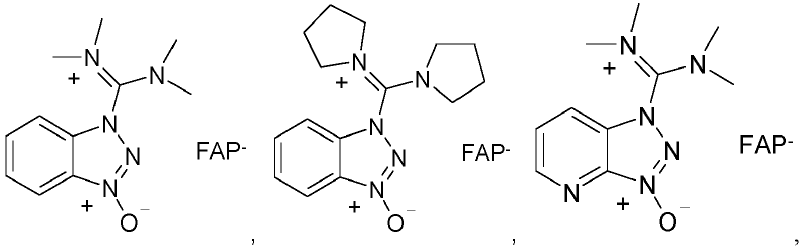

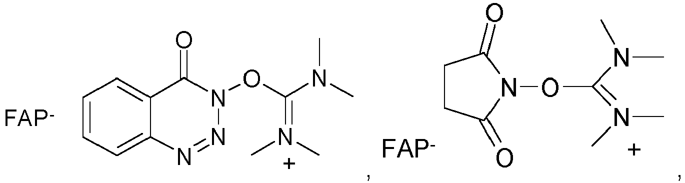

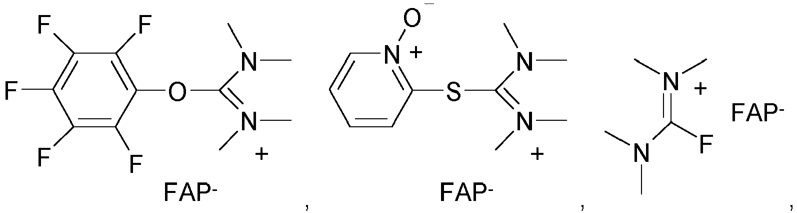

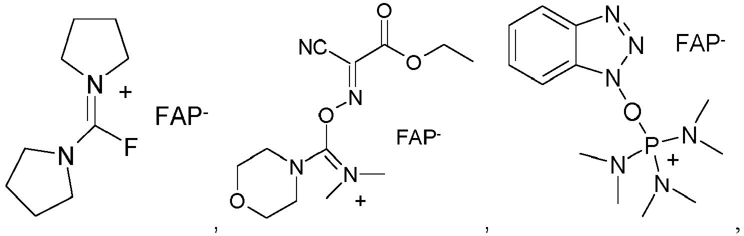

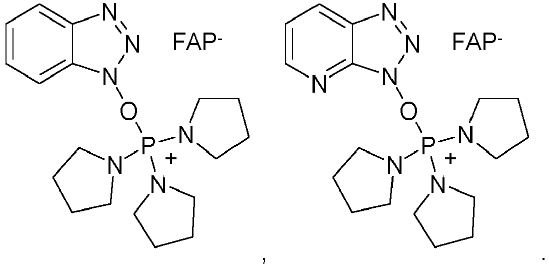

7. Compounds according to Claim 6, where FAP⁻ stands for [(C₂F₅)₃PF₃]⁻ or [(*n*-C₄F₉)₃PF₃]⁻.

## Revendications

1. Procédé de formation d'une liaison amide par la réaction d'un acide avec une amine primaire ou secondaire en présence d'une base à l'aide d'un réactif de liaison d'amide, **caractérisé en ce qu'**on utilise au moins un réactif de liaison d'amide contenant un anion de formule (I),
[P(CₙF₂ₙ₊₁)_{y}F_{6-y}]⁻ (I),
où
n représente à chaque occurrence, indépendamment, 1, 2, 3, 4, 5, 6, 7 ou 8 et
y représente 1, 2, 3 ou 4.

2. Procédé selon la revendication 1, dans lequel on utilise des réactifs de liaison d'amide contenant des anions de formule (I) où la variable n représente à chaque occurrence, indépendamment, 2, 3 ou 4.

3. Procédé selon la revendication 1 ou 2, dans lequel on utilise des réactifs de liaison d'amide contenant des anions de formule (I) choisis dans le groupe constitué par [(C₂F₅)₃PF₃]⁻, [(C₃F₇)₃PF₃]⁻, [(C₄F₉)₃PF₃]⁻, [(C₂F₅)₂PF₄]⁻, [(C₃F₇)₂PF₄]⁻, [(C₄F₉)₂PF₄]⁻, [(C₂F₅)PF₅]⁻, [(C₃F₇)PF₅]⁻ et [(C₄F₉)PF₅]⁻

4. Procédé selon l'une ou plusieurs parmi les revendications 1 à 3, **caractérisé en ce que** le cation du réactif de liaison d'amide est un cation uronium, thiouronium, guanidinium, aminium, carbonium, imidazolium ou phosphonium.

5. Procédé selon l'une ou plusieurs parmi les revendications 1 à 4, **caractérisé en ce qu'**une liaison peptidique est formée.

6. Composés issus du groupe suivant de composés, où FAP⁻ représente un anion de formule (I), tel que décrit selon la revendication 1 :

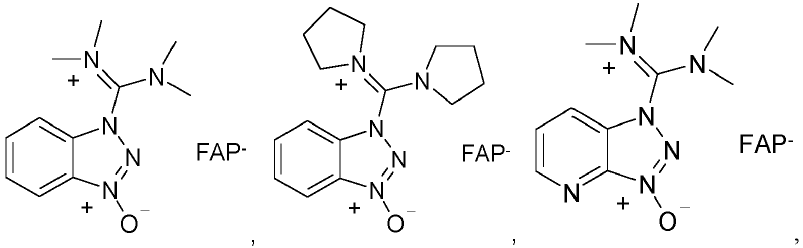

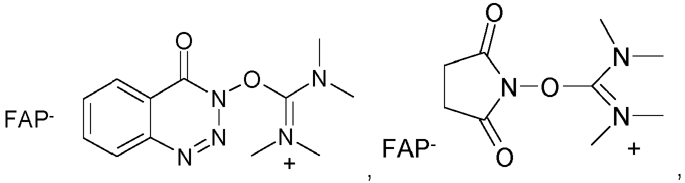

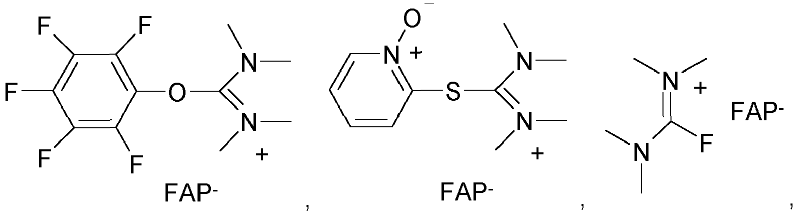

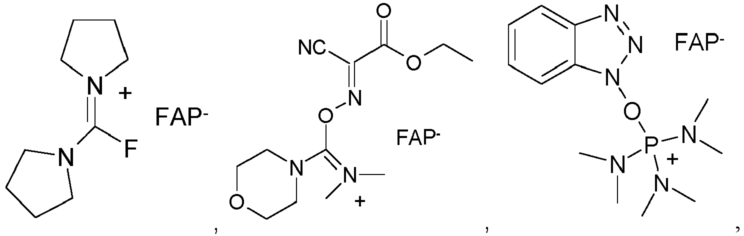

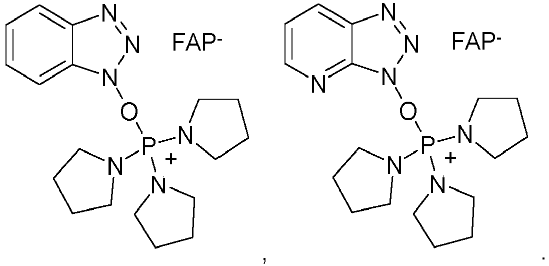

7. Composés selon la revendication 6, où FAP⁻ représente [(C₂F₅)₃PF₃]⁻ ou [(*n*-C₄F₉)₃PF₃]⁻.
